# EUROPEAN PATENT APPLICATION

(11) **EP 2 896 361 A1**
(43) Date of publication of application: **22.07.2015**
(21) Application number: 15151276.1
(22) Date of filing: 15.01.2015
(51) Int. Cl.: A61B 5/08, A61B 5/097, A61M 16/00

(54) **Accelerated closed-circuit wash-in of tracer gas using bolus injection in lung function testing**

(30) Priority: 17.01.2014 EP 14151599
(71) Applicant: Innovision Aps, 5620 Glamsbjerg (DK)
(72) Inventor: Clemensen, Peter Christian, 5000 Odense C (DK); Nielsen, Jørgen Grønlund, 5620 Glamsbjerg (DK)
(74) Representative: Zacco Denmark A/S

(57) **Abstract**

The present invention relates to methods aiming at fast equilibration in the lungs of inhaled tracer gases by inhalation of a bolus of gas mixture followed by rebreathing in a closed system.

Possible applications of the methods include inert gas rebreathing for fast wash-in of tracer gas for multiple-breath washout measurements to determine the lung clearance index (LCI) or other indices of ventilation inhomogeneity of the lungs or for pulmonary blood flow and lung volume determination by rebreathing.

Furthermore the present invention relates to a corresponding system.

## Description

### Field of the invention

Certain pulmonary function testing techniques require even distribution and optimal mixing of inhaled tracer gases and resident gas in the lungs prior to the measurement of respired gases for determination of pulmonary and/or cardiovascular parameters. The present invention relates to methods aiming at fast equilibration in the lungs of inhaled tracer gases by inhalation of a bolus of gas mixture followed by rebreathing in a closed system.

Possible applications of the methods include inert gas rebreathing for fast wash-in of tracer gas for multiple-breath washout measurements to determine the lung clearance index (LCI) or other indices of ventilation inhomogeneity of the lungs or for pulmonary blood flow and lung volume determination by rebreathing.

### Background

Ventilation inhomogeneity or ventilation distribution in the lungs may be assessed using the multiple-breath washout (MBW) technique performed by washing out a previously washed in exogenous inert tracer gas during tidal breathing of room air.

When using the MBW technique several indices of overall ventilation inhomogeneity can be calculated as sensitive measures of airway disease. They all reflect differences in specific ventilation between lung regions resulting in delayed washout of the tracer gas from poorly ventilated regions. One example is the lung clearance index (LCI), which represents the number of lung volume turnovers required to clear the lungs from the tracer gas. It is calculated as the cumulative expired volume required for clearing the gas from the lungs minus the number of washout breaths multiplied by external dead space, divided by the subject's functional residual capacity (FRC). FRC is the amount of air staying in the lungs after a normal expiration.

Different exogenous gases with low solubility in blood and tissues can be used, including sulfur hexafluoride (SF₆). The test subject is breathing through a mouthpiece or a face mask connected to a flowmeter, and a gas sampling tube is connected between the breathing assembly and a gas analyser. The tracer gas must first be washed in to obtain full equilibration (i.e. an even distribution in the lungs) before the washout can start. This can be achieved using an open-circuit method, which is the conventional method wherein a bias flow of tracer gas exceeding the peak inspiratory flow of the test subject is used. Alternatively, a closed-circuit method, inert gas rebreathing (IGR), can be used for wash-in of the tracer gas, resulting in substantially reduced wash-in time.

After wash-in, the washout should continue until the end-tidal tracer gas concentration has fallen below 1/40th of the starting concentration over three successive breaths.

Inert gas rebreathing is also a well-established non-invasive method for determination of pulmonary blood flow and functional residual capacity. In the absence of a significant intrapulmonary shunt the pulmonary blood flow is equal to the cardiac output, i.e. the amount of blood pumped by the left ventricle of the heart per unit time.

The method uses a mixture containing two inert tracer gases, a blood soluble (e.g. nitrous oxide, N₂O) and an insoluble (e.g. SF₆ as in MBW) compound. The subject inhales the gas mixture from a rebreathing bag and rebreathes the mixture in the closed rebreathing system until equilibration of the insoluble gas has been obtained for a few breaths. When the blood soluble gas comes in contact with the blood perfusing the lungs it is absorbed and therefore gradually disappears from the closed rebreathing system at a rate which is proportional to the blood flow.

The blood insoluble gas is used to determine the lung volume by dilution. The lung volume is an important variable in the relationship between the disappearance rate of the blood soluble gas and pulmonary blood flow. It is also used as a tracer to account for various basic assumptions of the underlying lung model.

Both when rebreathing for washing in tracer gas for the MBW method and when rebreathing for determining cardiac output and lung volume quick mixing within the lungs or between lungs and bag is desired. Factors affecting the wash-in time include ventilation inhomogeneity, incomplete emptying of the rebreathing bag, and dead space.

### Summary of the invention

The object of the present invention is to provide method(s) to achieve fast equilibration in the lungs of inhaled tracer gas(es) by inhalation of a bolus of gas mixture in order to deliver all or most tracer gas(es) in the first part of the first inspiration of a rebreathing test using a closed circuit.

This is accomplished by a method for obtaining fast equilibrium of at least one inert tracer gas in the lungs of a test subject prior to the measurement of respired gases for determination of pulmonary and/or cardiovascular parameters of the test subject, wherein said method is a rebreathing method using a system comprising a rebreathing valve assembly and a closed bag system comprising a rebreathing port, a rebreathing bag and optionally a carbon dioxide (CO₂) scrubber, wherein said closed bag system is filled with a volume of an initial gas mixture comprising said at least one inert tracer gas prior to a rebreathing period, said method comprising the steps of:
- inhalation of a bolus gas mixture, the bolus gas mixture comprising all or most of the inert tracer gas comprised in the closed bag system thereby delivering all or most inert tracer gas in a first part of the first inspiration of the rebreathing period,
- continued inhalation of a last part of the first inspiration of the rebreathing period of a part of the volume of the initial gas mixture containing none or a lower concentration of the inert tracer gas than that in the bolus gas mixture,
- subsequent exhalation by the test subject whereby said closed bag system is comprising an alternated gas mixture comprising said inert tracer gas in a lower concentration than that in the initial gas mixture, and
- rebreathing of the alternated gas mixture to wash-in the inert tracer gas until a constant concentration of the inert tracer gas in the lungs of the test subject is reached.

### Brief description of the drawings

In the following, preferred embodiments of the invention will be described referring to the figures, where;
figure 1 (prior art) is a schematic diagram illustrating a setup for inert gas rebreathing using a bag with tracer gas mixture, where the tracer gas is uniformly distributed in the bag as known in the art,
figure 2 (prior art) illustrates a curve from wash-in of tracer gas (SF₆) using conventional rebreathing,
figure 3 is a schematic diagram illustrating a setup for accelerated wash-in of tracer gas using bolus injection during rebreathing as used in conjunction with the disclosed invention,
figure 4 is an example comparing the wash-in of an inert tracer gas by bolus injection during rebreathing as used in conjunction with the disclosed invention (dashed curve) and by conventional rebreathing, where the tracer gas is uniformly distributed in the bag, as known in the art (solid curve), respectively. The simulation in the example is performed using the following input values: FRC = 2.0 l, dead space V_{D} = 0.2 l, bag volume V_{RB} = 1.0 l, tidal volume V_{T} = 0.85 l, and

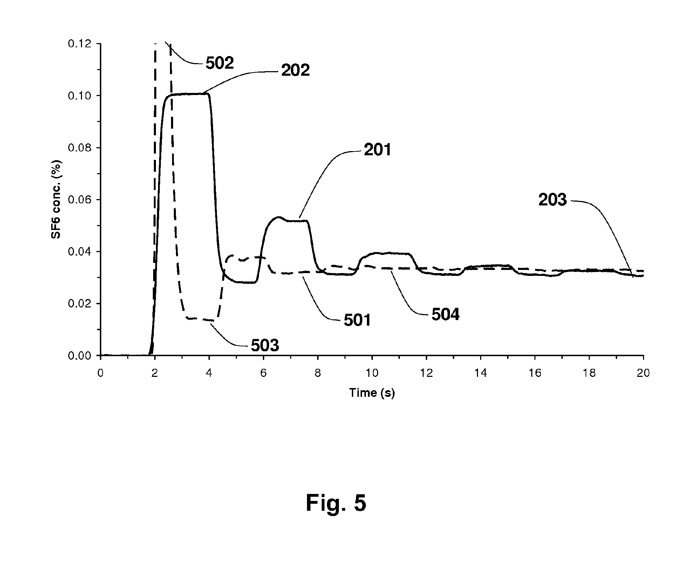

figure 5 is an example comparing experimental curves from wash-in of an inert tracer gas (SF₆) by bolus injection during rebreathing as used in conjunction with the disclosed invention (dashed curve) and by conventional rebreathing, where the tracer gas is uniformly distributed in the bag, as known in the art (solid curve), respectively. Bolus injection is obtained by filling a bolus of 10% of the bag volume containing 1 % SF₆ into the bag at the end of the filling procedure with a CO₂ scrubber placed in the bag port and with the bag upside down thereby obtaining an intended non-uniform initial mixture in the bag.

### Detailed description of the invention

The object of the present invention is to provide method(s) to achieve fast equilibration in the lungs of inhaled tracer gas(es) by inhalation of a bolus of gas mixture in order to deliver all or most tracer gas(es) in the first part of the first inspiration of a rebreathing test using a closed circuit.

This is accomplished by a method for obtaining fast equilibrium of at least one inert tracer gas in the lungs of a test subject prior to the measurement of respired gases for determination of pulmonary and/or cardiovascular parameters of the test subject, wherein said method is a rebreathing method using a system comprising a rebreathing valve assembly and a closed bag system comprising a rebreathing port, a rebreathing bag and optionally a carbon dioxide (CO₂) scrubber, wherein said closed bag system is filled with a volume of an initial gas mixture comprising said at least one inert tracer gas prior to a rebreathing period, said method comprising the steps of:
- inhalation of a bolus gas mixture, the bolus gas mixture comprising all or most of the inert tracer gas comprised in the closed bag system thereby delivering all or most inert tracer gas in a first part of the first inspiration of the rebreathing period,
- continued inhalation of a last part of the first inspiration of the rebreathing period of a part of the volume of the initial gas mixture containing none or a lower concentration of the inert tracer gas than that in the bolus gas mixture,
- the method may further comprise subsequent exhalation by the test subject whereby said closed bag system is comprising an alternated gas mixture comprising said inert tracer gas in a lower concentration than that in the initial gas mixture, and
- rebreathing of the alternated gas mixture to wash-in the inert tracer gas until a constant concentration of the inert tracer gas in the lungs of the test subject is reached.

The closed bag system is filled with a volume of an initial gas mixture comprising the at least one inert tracer gas where the average concentration of the inert tracer gas in the closed bag system before starting the rebreathing test will be lower than the concentration of the inert tracer gas in the part of the initial gas mixture comprising the bolus gas mixture and higher than the concentration of the inert tracer gas in the rest of the initial gas mixture in the closed bag system. This means that after the inhalation of the bolus gas mixture in a first part of the first inspiration of the rebreathing period, the average concentration of the inert tracer gas in the closed bag system will decrease. The average concentration of the inert tracer gas in the closed bag system will now be none or markedly lower than the average concentration of the inert tracer gas to start with and also markedly lower than the concentration in the inhaled bolus gas. When the test subject after the inhalation of the bolus gas mixture in a first part of the first inspiration of the rebreathing period continues the inhalation of a last part of the first inspiration - then the concentration of the inert tracer gas in the inhaled part of the volume of the initial gas mixture will be none or markedly lower than the avarage concentration of the inert tracer gas in the initial gas mixture in the closed bag system to start with and also lower than the concentration in the inhaled bolus gas. During the subsequent exhalation by the test subject the closed bag system comprises an alternated gas mixture comprising said inert tracer gas in a lower concentration than the average concentration of the inert tracer gas in the closed bag system before starting the rebreathing test. That is the alternated gas mixture comprises the inert tracer gas in a lower concentration than the avarage concentration in the initial gas mixture.

In one embodiment the bolus gas mixture comprising said inert tracer gas constitutes from about 5 to 50%, such as from 10 to 40%, such as from 10 to 20% or around 10% of the volume of the closed bag system. In one embodiment the bolus gas mixture comprising said inert tracer gas constitutes 10% of the volume of the closed bag system. Bolus injection can be obtained by filling a bolus of e.g. 10% of the bag volume into the bag at the end of the filling procedure with a CO₂ scrubber placed in the bag port and with the bag upside down. When the test subject is at rest, the volume of the bolus gas mixture could constitute approximately 10% of the volume of the closed bag system whereas if the test subject is performing some kind of exercise the volume of the bolus gas mixture could constitute approximately 20, 30, 40 or 50% of the volume of the closed bag system. In this context the bolus gas mixture is an oxygen enriched gas mixture.

The bolus gas mixture according to the present invention is the gas led from a gas reservoir 109 via a gas line 110 to the closed bag system. The volume of the bolus gas mixture filled into the closed bag system constitutes less than the total volume of the closed bag system. In one embodiment the bolus gas mixture comprises O₂ (oxygen) and one tracer gas, where the tracer gas is an insoluble gas such as SF₆. In one embodiment the bolus gas mixture comprises O₂ (oxygen) and two tracer gases, where the one tracer gas can be a blood soluble gas such as N₂O and the other tracer gas can be an insoluble gas such as SF₆.

The concentration of inert tracer gas is higher in the bolus gas mixture than in the rest of the gas mixture comprised in the closed bag system after filling. So when the closed bag system is filled it will comprise two phases, one comprising the bolus gas mixture and one comprising atmospheric air (ambient air) or a mixture of the two. In contrast, a closed bag system as known in the art would contain a homogeneous gas mixture.

In one embodiment the concentration of the insoluble gas such as SF₆ in the bolus gas mixture is from about 0.5 to 10%, such as from 1 to 10%, such as from 1 to 5%, such as around 1%.

In one embodiment the concentration of the blood soluble gas such as N₂O in the bolus gas mixture is from about 0.5 to 15%, such as from 1 to 10%, such as from 1 to 5%, such as around 5%.

In one embodiment where the bolus gas mixture is composed of 1% SF₆, balance O₂ and where 10% of the volume of the closed bag system is composed of the bolus gas mixture and the rest of the closed bag system is composed of ambient air, then the total fraction of SF₆ in the closed bag system is 0.1 %.

In general the rebreathing method is a more effective manoeuvre to obtain good mixing of the gases in the lungs compared to multiple-breath wash-in and by the above method an even faster and more effective manoeuvre to obtain good mixing of the gases is obtained.

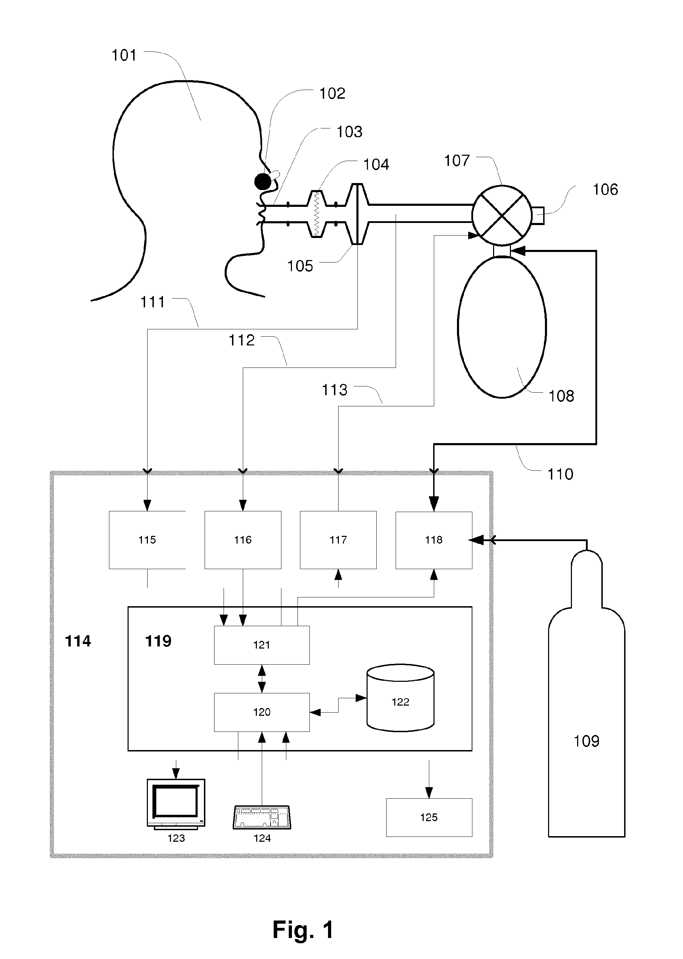

Figure 1 (prior art) is a schematic diagram illustrating a setup for inert gas rebreathing using a bag with tracer gas mixture as known in the art, where the tracer gas is uniformly distributed in the bag. A test subject 101 having the nose occluded with a nose clip 102 breathes through a mouthpiece 103, a bacterial filter 104, a respiratory flowmeter 105 and one port 106 of a rebreathing valve assembly 107. A rebreathing bag 108 is connected to the valve assembly and evacuated and prefilled with a uniform gas mixture from a gas reservoir 109 via a gas line 110. Flowmeter connection(s) 111 and a gas sample line 112 are also part of the setup.

To perform a rebreathing manoeuvre the valve assembly 107 is switched (e.g. automatically by controlling line 113) to allow the test subject 101 to inspire and rebreathe to and from the bag 108 for a certain amount of time until the valve assembly 107 is switched back again. The test subject 101 may use a face mask instead of nose clip 102 and mouthpiece 103. The control system 114 of the measuring apparatus consists of flowmeter electronics 115, at least one gas analyser 116, a valve control unit 117 (unless the valve assembly is manually driven) and a gas control unit 118 (unless the bag is prepared manually). A control unit 119 is also included, comprising a computing/processing unit (CPU) 120 with control interfaces 121, one or more program and data storage devices 122 and user interfaces for example comprising a display 123 and a keyboard, touch screen or similar input device 124. A data input/output module 125 may also be included.

The processing unit (CPU) can e.g. comprise processing means for determining LCI and FRC of the lungs of a test subject using the obtained fractional concentration of the insoluble tracer gas measured by the gas analyser and the gas flow measured by the flowmeter and associated flowmeter electronics. Also, the processing unit can e.g. comprise processing means for determining pulmonary blood flow and FRC based on insoluble and blood soluble tracer gas analysis.

Prior to the rebreathing manoeuvre the rebreathing bag is filled with a known volume of an inert tracer gas mixture which is well mixed in the bag. During the manoeuvre the test subject is breathing through the rebreathing valve assembly, which allows switching from breathing air to rebreathing the uniform inert tracer gas mixture from the bag and switching back again.

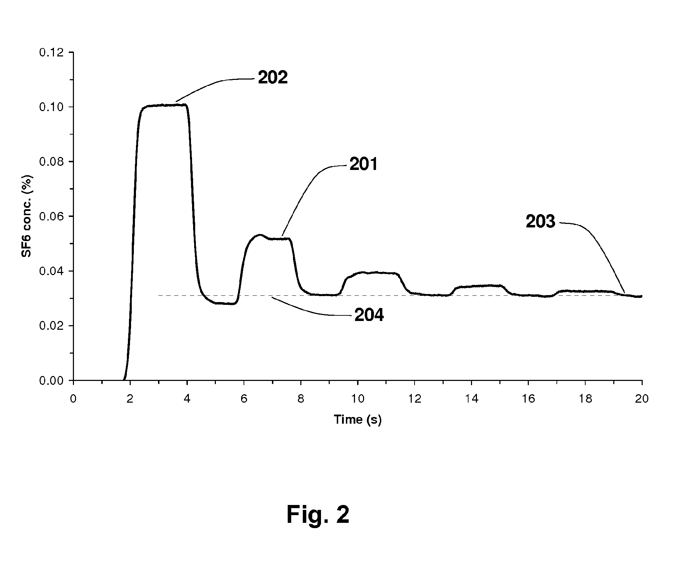

During the testing the concentrations of the inert tracer gases in the mixture are measured by a fast responding gas analyser 116. Hereby the concentration curves as illustrated later in figure 2 can be obtained. Instead of gas concentrations the gas analyser may equally well measure the partial pressures of the gases. The partial pressures can be obtained from the fractional concentrations of dry gas or any other measure of gas concentration or pressure using appropriate conversion factors as known in the art. Also the flow of the inhaled and/or exhaled air is measured by means of the flowmeter 115. These measurements are made continuously.

Figure 2 (prior art) illustrates a representative curve of an insoluble tracer gas 201 from a rebreathing test as known in the art. The concentration of the tracer gas increases to a plateau 202 corresponding to the initial concentration in the rebreathing bag during the first inspiration from the rebreathing bag and gradually equilibrates at a level 203 determined by the ratio of the rebreathing bag volume and the combined bag and lung volume. The equilibrium concentration is indicated by the dotted line 204.

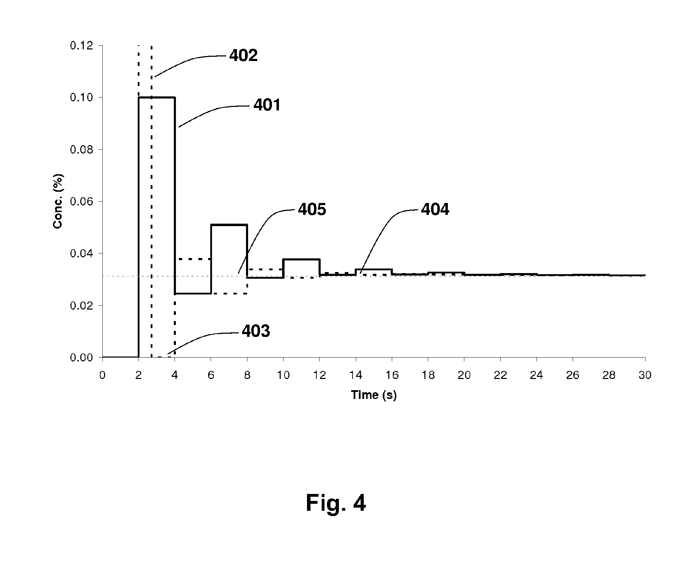

Computer simulations have shown that rebreathing using bolus injection results in faster equilibration of the inert gas than rebreathing using a mixed bag content, where the tracer gas is uniformly distributed in the bag, resulting in a significant reduction of total test time. See e.g. figure 4 which shows a comparison of inert gas rebreathing comprising the bolus gas mixture being injected into the closed bag system or directly injected (used as part of the invention) and rebreathing of a mixed bag content, where the tracer gas is uniformly distributed in the bag (prior art) using data from computer simulations.

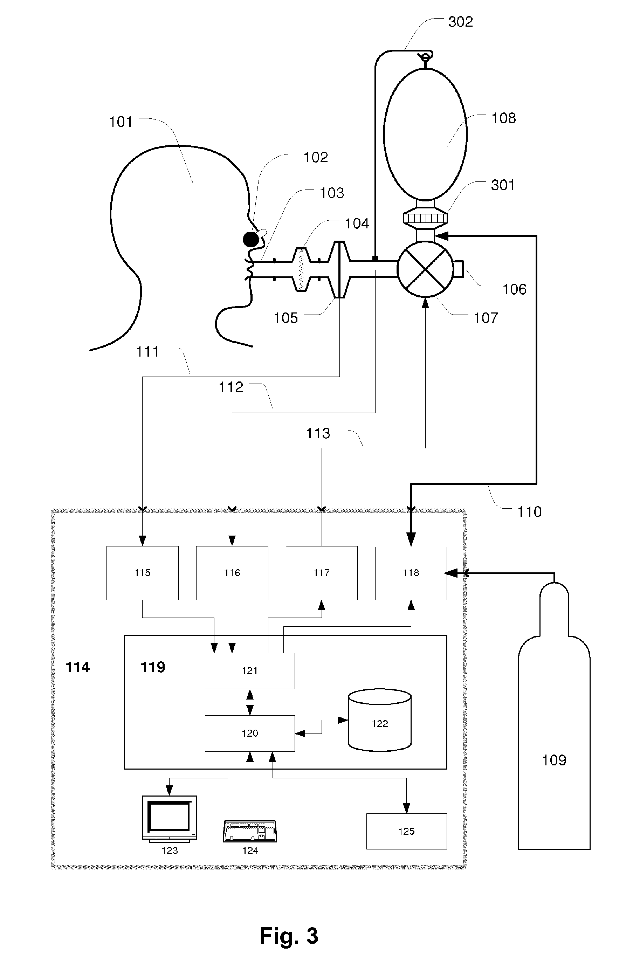

Figure 3 is a schematic diagram illustrating a setup for accelerated wash-in of tracer gas using bolus injection during rebreathing as used in conjunction with the disclosed inventions. This setup can be used e.g. for determination of FRC and ventilation distribution (LCI) by combined wash-in/washout of exogenous tracer gas or for determination of pulmonary blood flow and FRC using inert gas rebreathing only.

The setup includes the same constituent parts as in figure 1 with the addition of an effective CO₂ scrubber 301 and a holder 302 for upside-down suspension of the bag 108. The control system 114 of the measuring apparatus comprises a gas control unit 118 capable of sequentially delivering at least two gas mixtures through gas line 110, preferably one bolus of tracer gas mixture from a gas reservoir 109 and ambient air as the rest of the bag volume to obtain two phases of gas mixture in the bag 108.

Figure 4 is an example from a simulation comparing the wash-in of an inert insoluble tracer gas by conventional rebreathing, where the tracer gas is uniformly distributed in the bag as known in the art 401 (solid curve) and by bolus injection during rebreathing as used in conjunction with the disclosed invention 402 (dashed curve), respectively. The simulation is based on a two-compartment lung model and performed using the following input values: FRC = 2.0 l, dead space V_{D} = 0.2 l, bag volume V_{RB} = 1.0 l, and tidal volume V_{T} = 0.85 l. One compartment represents 40 % of the FRC and receives 60 % of the alveolar ventilation, whereas the other compartment represents 60 % of the FRC and receives 40 % of the alveolar ventilation and therefore is poorly ventilated. The volume of the bolus is 30 % of the initial bag volume, and the maximum tracer gas concentration during the first part of the first inspiration from the bag is 0.33 % (out of scale). When the bolus has been inspired and passed the point of gas sampling the tracer gas concentration goes to zero for the remainder of the first inspiration 403. The conventional rebreathing curve 401 was generated using the same lung parameters as above but using a constant concentration of 0.1 % tracer gas during the first inspiration from the bag. The simulation shows that when using bolus injection the tracer gas reaches equilibrium 404 much faster compared with conventional rebreathing. The equilibrium concentration is indicated by the dotted line 405.

Since equilibrium is reached faster by bolus injection the wash-in time and the total test time can be reduced substantially in MBW testing and the rebreathing manoeuvre significantly shortened when determining pulmonary blood flow and FRC by inert gas rebreathing.

Figure 5 is a typical example comparing experimental curves from wash-in of an inert tracer gas (SF₆) by bolus injection during rebreathing as used in conjunction with the disclosed invention 501 (dashed curve) and by conventional rebreathing as known in the art 201 (solid curve), respectively. Bolus injection is obtained by filling a bolus of 10 % of the bag volume into the bag at the end of the filling procedure with a CO₂ scrubber placed in the bag port and with the bag upside down as shown in one embodiment of the disclosed invention in figure 3. The maximum tracer gas concentration 502 during the first part of the first inspiration from the bag is 0.55 % (out of scale). When most of the bolus has been inspired and passed the point of gas sampling the tracer gas concentration goes down for the remainder of the first inspiration 503. In this example the concentration at the point of gas sampling does not reach zero as part of the bolus gas has diffused into the bottom of the rebreathing bag prior to the first inspiration. In this example the two phases of gas mixture will be partly mixed prior to the first inspiration.

The conventional rebreathing curve 201 is recorded using the same setup and bag volume but after complete mixing of the bolus volume and the rest of the bag volume prior to the test whereby a constant concentration of 0.1 % tracer gas is achieved during the first inspiration from the bag. SF₆ is used as tracer gas. The bolus gas mixture injected into the bag contains 1 % SF₆ and the bolus volume constitutes 10 % of the bag volume. The experimental curves confirm the observation from the simulation, i.e. when using the bolus technique to deliver the tracer gas, the tracer gas reaches equilibrium 504 much faster compared with conventional rebreathing (se 203 in figure 2 and 5).

There are different bolus gas delivery methodologies which can be employed to obtain reduced wash-in time:
In one embodiment the closed bag system is filled with the initial gas mixture comprising said at least one inert tracer gas by a sequential gas filling procedure prior to the rebreathing period, where the bolus gas mixture comprising said inert tracer gas is filled into the rebreathing bag during a last part of the sequential gas filling procedure thereby concentrating the inert tracer gas near the opening or neck of the rebreathing bag.

In one embodiment of the present invention the bolus gas is administered from gas reservoir 109 through gas line 110 during the last part of the bag filling procedure, thereby concentrating the tracer gas near the opening or neck of the bag 108. The first part of the filling procedure is used for delivering the other mixture not containing tracer gas into the bag 108. When the test subject 101 inspires the contents of the bag 108 all or most of the bolus volume containing the tracer gas will be inspired first whereby a larger proportion of the tracer gas will reach poorly ventilated regions of the lungs and thereby help to reduce wash-in time.

In one embodiment a CO₂ scrubber is added between the rebreathing port and the rebreathing bag, and where the closed bag system is filled with the initial gas mixture comprising said at least one inert tracer gas by a sequential gas filling procedure prior to the rebreathing period, where the bolus gas mixture comprising said inert tracer gas is entered into the closed bag system via the CO₂ scrubber during a last part of the sequential gas filling procedure thereby concentrating the inert tracer gas in the dead space of the CO₂ scrubber and near the opening or neck of the rebreathing bag.

In one embodiment the method comprises a CO₂ scrubber. When the test subject rebreathes in a closed system, an accumulation of CO₂ will take place in the system. This build up of CO₂ can be experienced as uncomfortable by the test subject even though there is no lack of oxygen (O₂). This build up of CO₂ can be reduced by implementing a CO₂ scrubber in the flow way e.g. between the rebreathing port and the rebreathing bag or by implementing a small parallel circuit using recirculation of gas through the CO₂ scrubber to avoid adding extra serial dead space.

In another embodiment of the present invention an effective CO₂ scrubber 301 is added between the rebreathing port of the valve assembly 107 and the bag 108. As above, the bolus gas is administered from gas reservoir 109 through gas line 110 connected to the bag port during the last part of the bag filling procedure, thereby concentrating the tracer gas in the dead space of the CO₂ scrubber 301 and near the opening or neck of the bag 108. The first part of the filling procedure is used for delivering the other mixture not containing tracer gas. The addition of the dead space of the CO₂ scrubber improves the separation of the bolus gas mixture and the other mixture not containing tracer gas, thereby reducing wash-in time further.

In one embodiment the closed bag system is filled with a volume of the initial gas mixture comprising said at least one inert tracer gas by a sequential gas filling procedure prior to the rebreathing period and wherein a first part of the sequential gas filling procedure is used for dispensing a mixture comprising a zero fraction of the total tracer gas volume into the rebreathing bag followed by filling the bolus gas mixture comprising said inert tracer gas during a last part of the sequential gas filling procedure.

In one embodiment the closed bag system is filled with a volume of the initial gas mixture comprising said at least one inert tracer gas by a sequential gas filling procedure prior to the rebreathing period and wherein a first part of the sequential gas filling procedure is used for dispensing a mixture comprising a non-zero fraction of the total tracer gas volume into the rebreathing bag followed by filling the bolus gas mixture comprising said inert tracer gas during a last part of the sequential gas filling procedure.

The first part of the sequential bag filling procedure can be used for dispensing a mixture containing a non-zero fraction of the total tracer gas volume into the bag 108 followed by a dose of pure bolus gas mixture. The aim is to obtain a fraction of tracer gas in the first part of the bag filling close to the estimated equilibrium concentration, e.g. calculated from the bag volume and predicted FRC and dead space. Consequently, the gas staying in the bag and dead space after the first inspiration from the bag would be closer to the equilibrium concentration with a possible further reduction of wash-in time as a result. The appropriate mixing of gas from gas reservoir 109 and room air is accomplished by the gas control unit 118.

In one embodiment all or part of the bolus gas mixture comprising said inert tracer gas is injected directly into the closed bag system at the bag port or closer to the test subject's mouth during a first part of the first inspiration of the rebreathing period.

This means that the method according to the invention in one embodiment is a method for obtaining fast equilibrium of at least one inert tracer gas in the lungs of a test subject prior to the measurement of respired gases for determination of pulmonary and/or cardiovascular parameters of the test subject, wherein said method is a rebreathing method using a system comprising a rebreathing valve assembly, and a closed bag system comprising a rebreathing port, a rebreathing bag and optionally a carbon dioxide (CO₂) scrubber, wherein said closed bag system can be filled with a volume of an initial gas mixture containing none or a low concentration of an inert tracer gas or be empty prior to a rebreathing period, said method comprising the steps of:
- inhalation of a bolus gas mixture, the bolus gas mixture comprising all or most of an inert tracer gas, the bolus gas mixture is injected directly into the closed bag system at the bag port or closer to the test subject's mouth during a first part of the first inspiration of the rebreathing period,
- optionally continued inhalation of a last part of the first inspiration of the rebreathing period of a part of the volume of the initial gas mixture containing none or a lower concentration of the inert tracer gas than that in the first part of the first inspiration of the rebreathing period,
- subsequent exhalation by the test subject whereby said closed bag system is comprising an alternated gas mixture comprising said inert tracer gas in a lower concentration than that in the first part of the first inspiration of the rebreathing period, and
- rebreathing of the alternated gas mixture to wash-in the inert tracer gas until a constant concentration of the inert tracer gas in the lungs of the test subject is reached.

The closed bag system is filled with a volume of an initial gas mixture comprising none or a low concentration of an inert tracer gas or be empty prior to a rebreathing period where the average concentration of the inert tracer gas in the closed bag system before starting the rebreathing test will be lower than the concentration of the inert tracer gas in the bolus gas mixture being injected directly into the closed bag system at the bag port or closer to the test subject's mouth during a first part of the first inspiration of the rebreathing period.

When the test subject after the inhalation of the bolus gas mixture in a first part of the first inspiration of the rebreathing period continues the inhalation of a last part of the first inspiration of a part of the volume of the initial gas mixture - then the concentration of the inert tracer gas in the inhaled part of the volume of the initial gas mixture will be none or lower than the concentration of the inert tracer gas in the inhaled bolus gas inhaled during the first part of the first inspiration of the rebreathing period. During the subsequent exhalation by the test subject the closed bag system comprises an alternated gas mixture comprising said inert tracer gas in a lower concentration than the concentration of the inert tracer gas in the inhaled bolus gas inhaled during the first part of the first inspiration of the rebreathing period.

If all or part of the bolus gas mixture comprising said inert tracer gas is injected directly into the closed bag system at the proximal side of the rebreathing valve assembly e.g. at the bag port or closer to the test subject's mouth, during a first part of the first inspiration, the closed bag system does not necessarily comprise a volume of an initial gas mixture containing none or a low concentration of an inert tracer gas - it can also just be empty. Then, after switching the rebreathing valve assembly to rebreathing mode, when the test subject subsequently exhales, the closed bag system will be filled with a tidal volume of that alternated gas that the test subject exhales and it is that gas that is subsequently rebreathed.

All or part of the bolus volume may be injected directly during the first part of the first inspiration from the rebreathing bag 108. The appropriate timing of the direct bolus gas injection is accomplished by the gas control unit 118, controlled by the control system of the measuring apparatus 114, which in turn receives information about the respiratory pattern from the flowmeter electronics 115 and/or the gas analyser 116, and also controls the valve control unit 117. The purpose of the direct bolus gas delivery system is the same as in the above embodiments, to reduce wash-in time.

In one embodiment the inert tracer gas is SF₆.

In one embodiment of the present invention the insoluble tracer gas is SF₆, and a holder 302 is applied for upside-down suspension of the bag 108. By placing the rebreathing bag upside-down the method takes advantage of the much higher density of SF₆ as SF₆ is a much heavier gas than atmospheric air.

SF₆ has become the gas of choice for measurement of LCI and is also well suited for lung volume determination in inert gas rebreathing. SF₆ has a molar mass of about 146 g/mol. For comparison, the molar mass of air, which is about 78 % nitrogen and 21 % oxygen, is approximately 30 g/mol. Due to the large molar mass of SF₆ its density is relatively high at room temperature and pressure. SF₆ has a density of 6.12 g/L at sea level conditions, which is considerably higher than the density of air (1.23 g/L). By suspending the bag 108 upside-down using the holder 302 and delivering the bolus gas mixture containing SF₆ from gas reservoir 109 through gas line 110 during the last part of the bag filling procedure, the SF₆ will settle in the low-lying areas of the CO₂ scrubber 301 (if used) and/or bag 108. Sequential bag filling and gravitational pull of SF₆ will therefore improve the separation of the bolus gas mixture and the other mixture not containing SF₆, thereby reducing wash-in time further as demonstrated in figure 5.

In one embodiment the bolus gas mixture comprising said at least one inert tracer gas is an oxygen enriched gas mixture. This is to avoid oxygen deficiency during the rebreathing manoeuvre.

In one embodiment the closed bag system is filled with a volume of the initial gas mixture comprising said at least one inert tracer gas corresponding to the test subject's tidal volume or up to 150% thereof. The test subject is connected to the bag via a rebreathing valve assembly. At the end of an expiration the valve is opened and the subject then inhales the initial gas mixture or part of the initial gas mixture from the bag and rebreathes the gas mixture in the closed rebreathing system until the inert tracer gas is well mixed in the lungs (see figure 3 for setup for wash-in/washout tests using inert gas rebreathing).

In one embodiment the gas analysis is performed by a sensitive gas analyser e.g. based on photoacoustic spectroscopy (PAS).

The present invention also discloses use for multiple-breath washout measurements to determine the lung clearance index (LCI) or other indices of ventilation inhomogeneity of the lungs or for pulmonary blood flow and lung volume determination by inert gas rebreathing.

The present invention also discloses a system adapted to obtain fast equilibrium of at least one inert tracer gas in the lungs of a test subject prior to the measurement of respired gases for determination of pulmonary and/or cardiovascular parameters of the test subject by a multiple-breath washout or an inert gas rebreathing method, said system comprising:
- a rebreathing valve assembly and a closed bag system comprising a rebreathing port, a rebreathing bag for the test subject to rebreathe to and from during a rebreathing period and optionally a CO₂ scrubber, wherein said closed bag system is filled with a volume of an initial gas mixture comprising said at least one inert tracer gas prior to the rebreathing period,
- a holder for upside-down suspension of the rebreathing bag,
- a gas control unit capable of sequentially delivering at least two gas mixtures through a gas line, preferably one bolus gas mixture comprising all or most of the inert tracer gas comprised in the closed bag system from a gas reservoir and ambient air as the rest of the volume of the initial gas mixture,
- at least one gas analyser for obtaining the fractional concentration of said inert tracer gas inhaled and exhaled by said test subject,
- a flowmeter for monitoring the gas flow inhaled and exhaled by said test subject, and
- -processing means for determining LCI or other indices of ventilation inhomogeneity of the lungs or for pulmonary blood flow and lung volume determination.

Hereby a system can be constructed that can achieve the same advantages as described above in relation to the method according to the present invention.

In one embodiment the bolus gas mixture comprising said inert tracer gas constitutes from about 5 to 50%, such as from 10 to 40%, such as from 10 to 20% or around 10% of the volume of the closed bag system.

The gas control unit of the system is capable of sequentially delivering at least two gas mixtures through a gas line. In one embodiment one of these two gas mixtures is the bolus gas mixture comprising all or most of the inert tracer gas comprised in the closed bag system where the bolus gas mixture comes from a gas reservoir. The other one of these two gas mixtures is ambient air filling the rest of the initial gas mixture volume. In one embodiment the first part of the filling procedure is used for delivering the other mixture not containing tracer gas. The second part of the filling procedure is used for delivering the bolus gas mixture.

The closed bag system is filled with a volume of an initial gas mixture comprising the at least one inert tracer gas where the average concentration of the inert tracer gas in the closed bag system before starting the rebreathing test will be lower than the concentration of the inert tracer gas in the part of the initial gas mixture comprising the bolus gas mixture and higher than the concentration of the inert tracer gas in the rest of the initial gas mixture in the closed bag system.

In one embodiment the closed bag system is filled with a volume of the initial gas mixture comprising said at least one inert tracer gas by a sequential gas filling procedure prior to the rebreathing period and wherein a first part of the sequential gas filling procedure is used for dispensing a mixture comprising a zero fraction of the total tracer gas volume into the rebreathing bag followed by filling the bolus gas mixture comprising said inert tracer gas during a last part of the sequential gas filling procedure.

In one embodiment the closed bag system is filled with a volume of the initial gas mixture comprising said at least one inert tracer gas by a sequential gas filling procedure prior to the rebreathing period and wherein a first part of the sequential gas filling procedure is used for dispensing a mixture comprising a non-zero fraction of the total tracer gas volume into the rebreathing bag followed by filling the bolus gas mixture comprising said inert tracer gas during a last part of the sequential gas filling procedure.

In one embodiment the first part of the sequential bag filling procedure can be used for dispensing a mixture containing a non-zero fraction of the total tracer gas volume into the bag 108 followed by a dose of pure bolus gas mixture. The aim is to obtain a fraction of tracer gas in the first part of the bag filling close to the estimated equilibrium concentration, e.g. calculated from the bag volume and predicted FRC and dead space. Consequently, the gas staying in the bag and dead space after the first inspiration from the bag would be closer to the equilibrium concentration with a possible further reduction of wash-in time as a result. The appropriate mixing of gas from gas reservoir 109 and room air is accomplished by the gas control unit 118.

The bolus gas mixture can be administered to the CO₂ scrubber from the gas reservoir through a gas line connected to the bag port during the last part of the bag filling procedure, thereby concentrating the tracer gas in the dead space of the CO₂ scrubber and near the opening or neck of the bag. The first part of the filling procedure is used for delivering the other mixture not containing tracer gas. The addition of the dead space of the CO₂ scrubber improves the separation of the bolus gas mixture and the other mixture not containing tracer gas, thereby reducing wash-in time further.

In one embodiment of the system the inert tracer gas is SF₆.

The bolus gas mixture according to the present invention is the gas led from a gas reservoir 109 via a gas line 110 to the closed bag system. In one embodiment the bolus gas mixture comprises O₂ (oxygen) and one tracer gas, where the tracer gas is an insoluble gas such as SF₆.

In one embodiment the bolus gas mixture comprises O₂ (oxygen) and two tracer gases, where the one tracer gas can be a blood soluble gas such as N₂O and the other tracer gas can be an insoluble gas such as SF₆.

In one embodiment the concentration of the insoluble gas such as SF₆ in the bolus gas mixture is from about 0.5 to 10%, such as from 1 to 10%, such as from 1 to 5%, such as around 1%.

In one embodiment the concentration of the blood soluble gas such as N₂O in the bolus gas mixture is from about 0.5 to 15%, such as from 1 to 10%, such as from 1 to 5%, such as around 5%.

In one embodiment where the bolus gas mixture is composed of 1% SF₆, balance O₂ and where 10% of the volume of the closed bag system is composed of the bolus gas mixture and the rest of the closed bag system is composed of ambient air, then the total fraction of SF₆ in the closed bag system is 0.1 %.

The concentration of inert tracer gas is higher in the bolus gas mixture than in the rest of the gas mixture comprised in the closed bag system after filling. So when the closed bag system is filled it will comprise two phases, one comprising the bolus gas mixture and one comprising atmospheric air (ambient air) or a mixture of the two. In contrast, a closed bag system as known in the art would contain a homogeneous gas mixture.

In one embodiment of the present invention the insoluble tracer gas is SF₆, and a holder 302 is applied for upside-down suspension of the bag 108. By placing the rebreathing bag upside-down the system takes advantage of the much higher density of SF₆ as SF₆ is a much heavier gas than atmospheric air. By suspending the bag 108 upside-down using the holder 302 and delivering the bolus gas containing SF₆ from gas reservoir 109 through gas line 110 during the last part of the bag filling procedure, the SF₆ will settle in the low-lying areas of the CO₂ scrubber 301 (if used) and/or bag 108. Sequential bag filling and gravitational pull of SF₆ will therefore improve the separation of the bolus gas mixture and the other mixture not containing SF₆, thereby reducing wash-in time further as demonstrated in figure 5.

SF₆ has very low solubility in blood and tissues and is a good choice when using infrared gas analysis techniques.

In one embodiment the system uses an oxygen enriched gas mixture comprising the inert tracer gas to avoid oxygen deficiency during the rebreathing manoeuvre.

The gas analyser could be any apparatus suitable for obtaining the fractional concentration of the inert tracer gas. Such apparatus could for instance be an infrared photoacoustic multi-gas analyser. In one embodiment of the invention the gas analysis is performed by a sensitive gas analyser with a high signal-to-noise ratio for example based on photoacoustic spectroscopy (PAS). The gas analyser can measure the end-tidal partial pressures or concentrations or a continuous partial pressure or concentration curve of the inert tracer gas.

The flowmeter could e.g. be a pneumotachometer, and is used to record the inspiratory and expiratory flows at the mouth. The pressure gradient measured is directly related to flow thus allowing a computer to derive a flow-curve measured in L/min.

The processing means for determining LCI and FRC of the lungs and/or pulmonary blood flow of a test subject is comprised in the control system of the measuring system.

In one embodiment the system comprises a CO₂ scrubber. When the test subject rebreathes in a closed system, an accumulation of CO₂ will take place in the system. This build up of CO₂ can be experienced as uncomfortable by the test subject even though there is no lack of oxygen (O₂). This build up of CO₂ can be reduced by implementing a CO₂ scrubber in the flow way e.g. between the rebreathing port and the rebreathing bag or by implementing a small parallel circuit using recirculation of gas through the CO₂ scrubber to avoid adding extra serial dead space. In one embodiment the CO₂ scrubber is added between the rebreathing port of the valve assembly and the bag. As above, the bolus gas mixture can be administered to the CO₂ scrubber from the gas reservoir through gas line connected to the bag port during the last part of the bag filling procedure, thereby concentrating the tracer gas in the dead space of the CO₂ scrubber and near the opening or neck of the bag. The first part of the filling procedure is used for delivering the other mixture not containing tracer gas. The addition of the dead space of the CO₂ scrubber improves the separation of the bolus gas mixture and the other mixture not containing tracer gas, thereby reducing wash-in time further.

In one embodiment the system comprises a small cylinder for containing the inert tracer gas mixture. Since the system has incorporated a sensitive gas analyser with a high signal-to-noise ratio and because rebreathing is the method of choice instead of open-circuit wash-in, a much smaller amount of inert tracer gas is necessary for performing the test. The small cylinder contains a high concentration of inert gas meaning that approx. only 1/10^{th} of the inhaled gas comes from the cylinder, the rest is atmospheric air or oxygen. The system can in one embodiment also comprise means to dilute the inert tracer gas mixture with air or oxygen. By using this dilution principle a much more compact system is obtained.

The processing means is comprised in the control system of the measuring system.

All descriptions throughout this document are made using fractional concentrations of dry inert gas. Fractional concentrations can be replaced by partial pressures using appropriate conversion factors as known in the art.

Volumes can be expressed at different gas conditions using appropriate conversion factors as known in the art.

It should be noted that the above-mentioned means of implementation illustrate rather than limit the invention, and that those skilled in the art will be able to suggest many alternative means of implementation without departing from the scope of the appended claims. Rather, the words used in the specification are words of description rather than limitation, and it is understood that various changes may be made without departing from the scope of the invention. The word 'comprising' does not exclude the presence of other elements or steps than those listed in a claim. The invention can be implemented by means of hardware and software comprising several distinct elements, and by means of a suitably programmed computer. In a device claim enumerating several means, several of these means can be implemented by one and the same item of hardware or software. The mere fact that certain measures are recited in mutually different dependent claims does not indicate that a combination of these measures cannot be used to advantage.

## Claims

1. A method for obtaining fast equilibrium of at least one inert tracer gas in the lungs of a test subject prior to the measurement of respired gases for determination of pulmonary and/or cardiovascular parameters of the test subject, wherein said method is a rebreathing method using a system comprising a rebreathing valve assembly and a closed bag system comprising a rebreathing port, a rebreathing bag and optionally a carbon dioxide (CO₂) scrubber, wherein said closed bag system is filled with a volume of an initial gas mixture comprising said at least one inert tracer gas prior to a rebreathing period, said method comprising the steps of:
- inhalation of a bolus gas mixture, the bolus gas mixture comprising all or most of the inert tracer gas comprised in the closed bag system thereby delivering all or most inert tracer gas in a first part of the first inspiration of the rebreathing period,
- continued inhalation of a last part of the first inspiration of the rebreathing period of a part of the volume of the initial gas mixture containing none or a lower concentration of the inert tracer gas than that in the bolus gas mixture,
- subsequent exhalation by the test subject whereby said closed bag system is comprising an alternated gas mixture comprising said inert tracer gas in a lower concentration than that in the initial gas mixture, and
- rebreathing of the alternated gas mixture to wash-in the inert tracer gas until a constant concentration of the inert tracer gas in the lungs of the test subject is reached.

2. A method according to claim 1 wherein the bolus gas mixture comprising said inert tracer gas constitutes from about 5 to 50%, such as from 10 to 40%, such as from 10 to 20% or around 10% of the volume of the closed bag system.

3. A method according to claim 1 or 2 where the closed bag system is filled with the initial gas mixture comprising said at least one inert tracer gas by a sequential gas filling procedure prior to the rebreathing period, where the bolus gas mixture comprising said inert tracer gas is filled into the rebreathing bag during a last part of the sequential gas filling procedure thereby concentrating the inert tracer gas near the opening or neck of the rebreathing bag.

4. A method according to claim 1 or 2 where a CO₂ scrubber is added between the rebreathing port and the rebreathing bag, and where the closed bag system is filled with the initial gas mixture comprising said at least one inert tracer gas by a sequential gas filling procedure prior to the rebreathing period, where the bolus gas mixture comprising said inert tracer gas is entered into the closed bag system via the CO₂ scrubber during a last part of the sequential gas filling procedure thereby concentrating the inert tracer gas in the dead space of the CO₂ scrubber and near the opening or neck of the rebreathing bag.

5. A method according to any of the preceding claims where the closed bag system is filled with the initial gas mixture comprising said at least one inert tracer gas by a sequential gas filling procedure prior to the rebreathing period and wherein a first part of the sequential gas filling procedure is used for dispensing a mixture comprising a zero fraction of the total tracer gas volume into the rebreathing bag followed by filling the bolus gas mixture comprising said inert tracer gas during a last part of the sequential gas filling procedure.

6. A method according to any of the preceding claims where the closed bag system is filled with the initial gas mixture comprising said at least one inert tracer gas by a sequential gas filling procedure prior to the rebreathing period and wherein a first part of the sequential gas filling procedure is used for dispensing a mixture comprising a non-zero fraction of the total tracer gas volume into the rebreathing bag followed by filling the bolus gas mixture comprising said inert tracer gas during a last part of the sequential gas filling procedure.

7. A method according to any of the preceding claims wherein all or part of the bolus gas mixture comprising said inert tracer gas is injected directly into the closed bag system at the bag port or closer to the test subject's mouth during a first part of the first inspiration of the rebreathing period.

8. A method according to any of the preceding claims wherein the inert tracer gas is SF₆.

9. A method according to any of the preceding claims where the bolus gas mixture comprising said at least one inert tracer gas is an oxygen enriched gas mixture.

10. A method according to any of the preceding claims where the closed bag system is filled with a volume of the initial gas mixture comprising said at least one inert tracer gas corresponding to the test subject's tidal volume or up to 150% thereof.

11. A method according to any of the preceding claims where gas analysis is performed by a sensitive gas analyser e.g. based on photoacoustic spectroscopy (PAS).

12. Use of a method according to claims 1 to 11, for multiple-breath washout measurements to determine the lung clearance index (LCI) or other indices of ventilation inhomogeneity of the lungs or for pulmonary blood flow and lung volume determination by inert gas rebreathing.

13. A system adapted to obtain fast equilibrium of at least one inert tracer gas in the lungs of a test subject prior to the measurement of respired gases for determination of pulmonary and/or cardiovascular parameters of the test subject by a multiple-breath washout or an inert gas rebreathing method, said system comprising:
- a rebreathing valve assembly and a closed bag system comprising a rebreathing port, a rebreathing bag for the test subject to rebreathe to and from during a rebreathing period and optionally a CO₂ scrubber, wherein said closed bag system is filled with a volume of an initial gas mixture comprising said at least one inert tracer gas prior to the rebreathing period,
- a holder for upside-down suspension of the rebreathing bag,
- a gas control unit capable of sequentially delivering at least two gas mixtures through a gas line, preferably one bolus gas mixture comprising all or most of the inert tracer gas comprised in the closed bag system from a gas reservoir and ambient air as the rest of the volume of the initial gas mixture,
- at least one gas analyser for obtaining the fractional concentration of said inert tracer gas inhaled and exhaled by said test subject,
- a flowmeter for monitoring the gas flow inhaled and exhaled by said test subject, and
- -processing means for determining LCI or other indices of ventilation inhomogeneity of the lungs or for pulmonary blood flow and lung volume determination.

14. A system according to claim 13 or 14 where the inert tracer gas is SF₆.

15. A system according to any of claims 13 to 14 where the gas analysis is performed by a sensitive gas analyser e.g. based on photoacoustic spectroscopy (PAS).
